# EUROPEAN PATENT APPLICATION

(11) **EP 4 813 364 A1**
(43) Date of publication of application: **30.09.2026**
(21) Application number: 25166898.4
(22) Date of filing: 28.03.2025
(51) Int. Cl.: A61F 13/15, D04H 1/732, D04H 1/736, G01N 21/89

(54) **INLINE INSPECTION AND CLEANING SYSTEM AND METHOD FOR FORAMINOUS STRUCTURES SUCH AS MASTER BELTS**

(71) Applicant: COAX Technologies S.r.l., 26010 Monte Cremasco Cremona (IT)
(72) Inventor: ZAMPOLLO, Fabio, 26013 Crema (IT)
(74) Representative: Plischke, Manfred

(57) **Abstract**

The present invention relates to a contact free image inspection system and a control and cleaning system for a foraminous structure, such as a master belt in the manufacturing of fibrous webs such as may be suitable for being used for absorbent articles, such as baby diapers, adult incontinence articles, but especially bed pads, meat pads or the like. In particular, the present invention relates to an effective inspection, control, and cleaning system in the manufacturing of air-laid webs, which can be directly fed into further article manufacturing converter lines.

## Description

### Field of the Invention

The present invention relates to a contact free image inspection system and a control and cleaning system for a foraminous structure, such as a master belt in the manufacturing of fibrous webs such as may be suitable for being used for absorbent articles, such as baby diapers, adult incontinence articles, but especially bed pads, meat pads or the like. In particular, the present invention relates to an effective inspection, control, and cleaning system in the manufacturing of air-laid webs, which can be directly fed into further article manufacturing converter lines.

### Background of the invention

Fibrous webs are currently produced by a number of different processes from fibers, optionally comprising particles. The fibers may be natural based, such as cellulosic fibers, or man-made natural material-based fibers, such as viscose / rayon or mineral based ones, or synthetic, typically polymeric based materials. For dry- or air-laying processes, the fibers or filaments forming fibers may be formed in-situ, such as in well-known processes as spunbonding or meltblowing, or may be pre-formed, such as when short fibers, such as cellulosic fibers, are provided e.g., in bale or roll form, and are separated from each other before being deposited on a an essentially continuously moving collecting surface, thereby forming a fibrous mat or web as will be moved from the collecting surface to further processing steps, such as consolidation. Typically, the collecting surface is of a foraminous element, and the lay-down is supported by air sucked through the foraminous element by a vacuum source. In many systems, the collection surface is a plane, and the foraminous element is a continuously moving collector, such as a belt, which exhibits a permeability allowing the air to pass through but retaining the material deposited thereon. In any of these cases, the deposition of the deposited material shall be as homogeneous and even as much as possible or according to a predetermined pattern, be this by adjusting the machine and cross-machine directional distribution of the material, optionally time dependent, or by employing a collector with regions of varying permeability.

However, even for carefully balanced suction, some of the deposited material, such as debris or break-up of the fibrous or particulate material, may penetrate through the openings of the foraminous collector. Such material may then be caught in further downstream air-cleaning systems, such as filters etc. But some of the deposited material may remain in the openings of the collector, thereby clogging these. This is highly undesirable, as this not only increases the requirements for the vacuum system to still maintain good suction, but it may also result in non-homogeneous material distribution, or deviations from the target lay-down pattern.

Henceforth, there exist various approaches for cleaning the collector, such as tapping, brushing, or by pressurized air. However, such cleaning processes are applied indiscriminately over the full width of the collector, as well as permanently, or operator induced, often based upon an overall parameter such as an increased pressure differential across the collector. Thereby the collector may be unduly mechanically stressed, thereby increasing wear and reducing life time, or significant energy, such as for the cleaning air blower, may be wasted.

Henceforth, it is an object of the present invention to provide and to operate a system for efficiently cleaning a collector system, such as a belt by employing a contact free control system and combining this with efficient cleaning operation.

Contact free inspection systems are known in the art for other applications, see e.g., EP2726039, wherein the positioning of various elements of an absorbent article are controlled by an image control system, or WO2019229273A1 (CCSol) describing a manufacturing system for absorbent structures with a detection system to quantitatively determine and control at high speeds the amount of superabsorbent polymeric (SAP) particles in an SAP/fluff mix by employing a video analysis system using radio frequency analysis, as generally known from WO2018/039535 (Accusentry) or WO2018/204724 (Alcatera), determining the amount of SAP and fluff in the analyzed sample. In EP3943917B1 (Fameccanica), a method for the in-line analysis of a composite product with SAP and fluff is described, wherein a hyperspectral sensor is used to acquire images of samples of target materials that are part of the composite product, in order to perform an in-line optical inspection at process speed. Other image analysis systems are available on a commercial basis, such as from Cognex Corp., MA, USA, or Mitsubishi Electric Corp., JP. However, such systems provide tool for enhancing quality of the formed web, such as uniformity or occurrence of nits or spots, but these do not address the problem of clogged collector systems.

### Summary of the Invention

Henceforth, in a first aspect, the present invention is an equipment for inspecting and cleaning a foraminous collector, preferably a foraminous belt for collecting fibrous and optionally particulate material. The equipment comprises
1) an air-laying apparatus exhibiting machine machine-direction MD, cross-machine direction CD and a z- or height direction perpendicular to MD and CD, the air-laying apparatus comprising
   1a) a first material supply unit, preferably a fiber supply unit for supplying fibers or fiber forming filaments, and optionally a second material supply unit, preferably a particulate material supply unit;
   1b) a material deposition unit, preferably a forming box, defining a deposition region;
2) a material collection unit, comprising
   2a) a closed loop air permeable collector system, comprising
   2a1) a continuously moving foraminous collector, preferably a foraminous belt;
   2a1i) comprising a material receiving surface and an opposite surface,
   2a1ii) and moving machine directionally through the material deposition region;
   2a2) the collector system comprising a set of drive and guide rolls adapted
   2a2i) to move the collector in a continuous closed loop along the processing direction of the collector at a collector speed, preferably monitored by a collector speed detector, and
   2a2ii) to transfer a web formed in the material deposition region on the collector to a further downstream processing unit;
   2b) a vacuum suction unit positioned in the fiber deposition region opposite to the material deposition unit and being adapted to provide an air flow through the collector from the material receiving surface to the opposite surface;
   2c) optionally a compaction unit for compacting the web;
3) a collector cleaning device comprising
   3a) at least one air blast system adapted to blow air through the air permeable collector in a direction from the opposite surface towards the material receiving surface;
   3b) an air blast system control device adapted to adjust time and CD-position of air blown through the collector;
4) a contact free inspection unit comprising
   4a) electromagnetic wave (EMW) receiver unit, comprising
   4a1) at least one EMW detector, which is
   4a1i) arranged
      - over at least one of the surfaces (1511, 1519) of the collector in a line or a 2D-matrix over the collector,
         or
      - movably cross-directionally over at least one of the surfaces (1511, 1519) of the collector;
   4a2) wherein the at least one EMW detector being adapted
   4a2i) to receive EMWs reflected from the surface (1511, 1519) of or penetrating through the collector, and
      - 4a2ii) to convert EMWs in the range of from 10 Hz to 1019Hz, preferably in the range
      - of visible light of about 1014 to 1015 Hz,
      - or of 1011 to 1012 Hz,
      - or of 3x103 Hz to 3x109 Hz,
      into an electric signal depending on the transparency or reflection of the collector;
   4b) optionally at least one EMW emitter unit, providing EMWs detectable by the detector(s);
   4c) a data processing unit adapted to convert the electric signal of the inspection system into signals for adjusting at least one of the processing parameters of the collector cleaning device by comparing the electric signal to a predetermined signal matrix.

The collector cleaning device and the EMW receiver unit are positioned in a region that is free of the web formed on the collector, and the air blast system of the collector cleaning device is adapted to provide air blasts upon activation of the air blast control device by the data processing unit, wherein the air blasts are discrete over time and position relative to the collector.

The detector may be oriented towards the receiving surface of the collection.

The cleaning system may comprise a multiplicity of cross-directionally arranged valves with nozzle units, which are adapted to be individually addressable. Alternatively, the cleaning system may comprise a valve with nozzle unit which is adapted to be cross-directionally movable.

In a second aspect, the present invention is process for inspecting and cleaning a foraminous collector, preferably a foraminous belt for collecting fibrous and optionally particulate material by operating such an equipment.

The process comprises the steps of
B) inspecting the collector in the inspection region by the contact-free inspection unit by receiving EMWs by the at least one receiver unit, whereby the inspecting comprising the steps of
   B1) receiving a time and position depending signal as a function of the collector's reflection or transparency for the EMWs,
   B2) transmitting the signal to the data processing unit;
C) analysing the signal in the data processing unit
C1) by comparing to a pre-determined set of collector's transparency or reflection parameters,
C2) and determining the position of an off-target collector region of the collector;
D) providing a signal for actuating the collector cleaning device depending on the time and position dependent variations;
E) moving the off-target collector region towards the collector cleaning device;
G) actuating the collector cleaning device so as to create an air blast at a time and position corresponding to the off-target collector region,
   wherein steps B) to G) are executed in a continuous operation.

In a third aspect, the present invention is a computer implemented method to control the cleaning of a web forming system comprising a movable foraminous material collector comprising the recuring steps of
- creating an image of the collector, which is mapping a pattern of reflected or penetrated EMWs;
- comparing the image to a predetermined image map of the collector and determining a deviation pattern, mapping the deviations cross-directionally and along the collector length by taking into account the speed of the collector;
- calculating when and where the air blast system needs to be actuated to create a cleaning air blast;
- actuating the air blast system.

### Brief description of the drawings

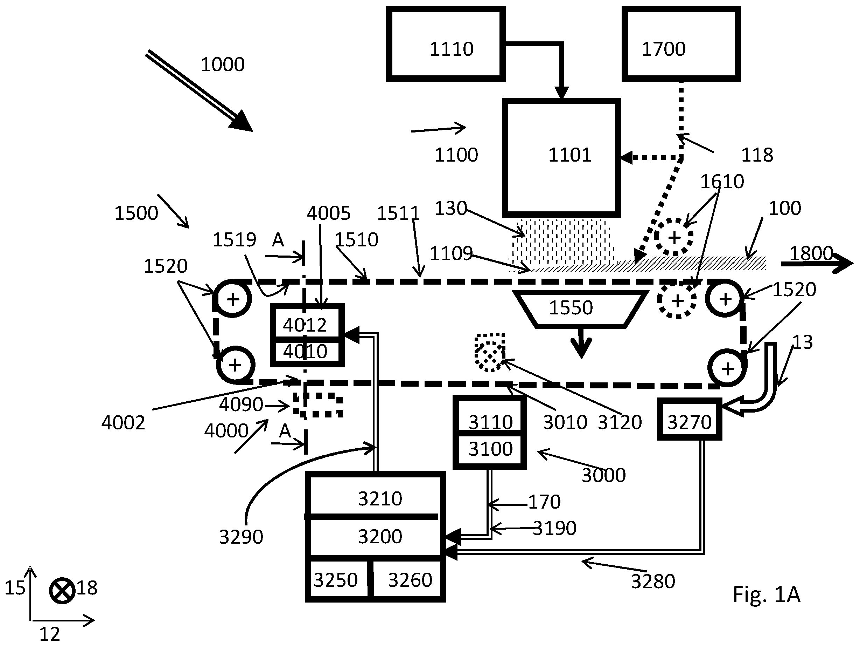

Fig. 1 A depicts a general layout of an air-laying system comprising the inspection and control elements according to the present invention.

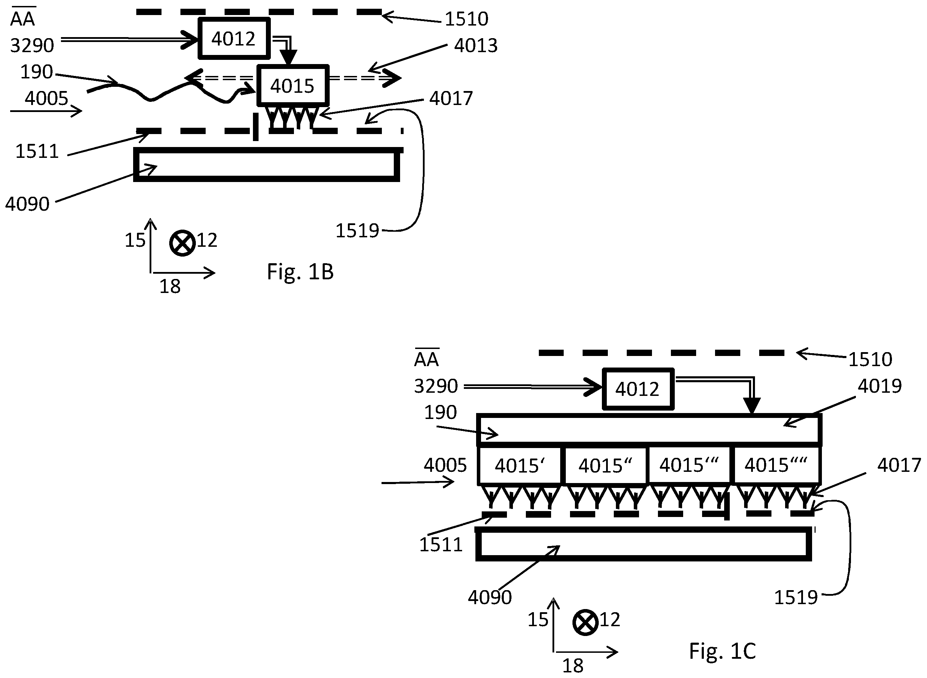

Fig. 1B and C depict specific execution of the cleaning system for the collector belt according to the present invention.

The figures are schematic only, and not to scale. Same numerals refer to same or equivalent features or elements, single (') or multiple (", '", ...) apostrophes indicate duplicate features, such a left and right or front and back, etc..

### Detailed Description

Thus, the present invention is an equipment and the corresponding methods for inspecting and cleaning foraminous structures onto which particulate material, especially short fibers, such as cellulosic fibers optionally intermixed with particles, such as superabsorbent polymer (SAP) particles, are deposited by a dry- or air-laying process, by which filamentary and/or particulate material suspended in an air stream are directed towards the foraminous structure, where the material is hold back by the foraminous structure whist the air is sucked therethrough by means of a vacuum applied opposite of the deposition surface of the foraminous structure. Without limitation, this may be employed in the manufacturing of fibrous webs, such as by air-laying, spunbonding or meltblowing. A particular application relates to the forming of absorbent structures, as may be absorbent cores, as well as to absorbent articles comprising such structures or cores, such as feminine napkins, panty liners, diapers, diaper pants, adult incontinence, bed sheets, puppy pads, and other products incorporating the use of absorbent structures or cores.

Fibrous structures may comprise fibers, such as - without limitation - from cellulosic material, be these manmade (like viscose / rayon) or natural, especially pulp fibers such as cellulosic treated or untreated pulp, or eucalyptus pulp, or from synthetic polymers, such as thermoplastic materials like polyolefins, or polyester, or polylactic acid.

The fibrous webs may exhibit basis weights of more than about 1 g/m², or more than about 10 g/m², or more than about 100 g/m², but typically less than about 1000 g/m².

The fibrous webs may be homogeneous, or may comprise a mixture of fibers, or may be of multiple sub-layers, as may be of the same material, or a different one.

The fibrous web may comprise particles, such as superabsorbent polymer particles, hereinafter also referred to as "SAP" particles, such as well-known to a person skilled in the art of disposable absorbent articles.-The amount of particles in the fibrous structure may be more than about 10 w-% or more than 40 w-% or more than about 60 w-%, or more than about 90 w-%, but typically less than about 99%. The particles may be with a particle size of more than about 45 µm and less than about 2000 µm, often less than 1200 µm or less than about 800 µm. However, the SAP material may also be in other forms, such as a fiber, such as available from Technical Absorbents, UK, or even in foamed form.

For the manufacturing of such fibrous structures, the fibers are typically individualized, suspended in air, and optionally with added particles deposited onto a foraminous structure, to which the air flow with fibers and particles, if present, is directed, so as to allow deposition and forming of a fibrous web. The foraminous structure may be a flexible continuous belt with an apertured surface, which allows the suspension air to pass through, but retains the fibers and/or particles on its surface in a deposition region. To this end, the opening in the apertured surface exhibit a balanced size, depending on the deposited material. If the openings are too small, the air flow is overly restricted, and excessive energy is required. If the openings are too large, too much of the fibers or particles are passing through and are either lost or can be recovered in costly operations, such as air filtering. However, during operation, not only the foraminous structure may be damaged, but the fibers and/or particles may deposit not only onto the foraminous structure, but also within the openings, e.g., after breaking up during previous operations, or simply being off-size. **In** order to remove such clogging, conventional methods typically apply continuous tapping or brushing or blowing air across the full width of the foraminous structure, optionally in pre-set intervals, or operator initiated, e.g., upon monitoring of the pressure drop. However, tapping and brushing typically lack efficiency, as the clogging is incompletely removed. Air-blowing is considered to provide efficient removal of the clogging; however, it requires significant amounts of pressurized air, which comes at significant energy cost.

Thus, in a first aspect, the present invention is a system for inspecting and cleaning foraminous structures, as is suitable for the manufacturing of fibrous webs, e.g., for absorbent structures, cores or articles.

**In** a second, process related aspect of the present invention, the foraminous structure is inspected by an electromagnetic waves inspection system positioned outside of the deposition region in an inspection region which is free of the formed web, and the resulting image map of the foraminous structure is compared with a predetermined target map. Upon this comparison, a cleaning step is initiated to be only applied in regions of the foraminous structure where clogging occurred. To this end, compressed air is selectively blown in a counter current direction through the foraminous structure in a cleaning region outside of the deposition region and the web transport region, thus being free of the formed web, thereby removing particulate material, i.e., fibers or particles, from the openings of the foraminous structure. Further, a receptacle may collect the material removed from the foraminous structure. In case that the deviation map indicates excessive deviations, the total lay-down process may be stopped.

In a third aspect, the present invention is computer implemented method of inspecting and cleaning foraminous structures.

As will be discussed in more detail herein below, it is an important aspect of the present invention that the system comprises or the methods utilize a particular detection system that applies electro-magnetic sensing technology to receive electromagnetic waves (or "EM waves") in the frequency range of from 10 Hz to 10¹⁹Hz, preferably in the ranges
- of visible light of about 10¹⁴ to 10¹⁵ Hz, with systems available from Cognex Inc, Natick, MA, USA, or Mitsubishi Electric Europe B.V., Germany, under the trade designation Line Scan Bar;
- or of 10¹¹ to 10¹² Hz, and especially in the range of more than 100 GHz, or more than 200 GHz or more than 300 GHz, but less than 900 GHz, preferably less than 600 GHz, for which suitable systems have been developed e.g., by Alcatera LLC, Los Angeles, USA, or Terasense Group Inc. USA;
- or of 3x10³ Hz to 3x10⁹ Hz, for which suitable systems have been developed by Accusentry, Marietta, GA, USA.

The signals of the EM wave sensors provide an actual map image of the foraminous structure, and a data processing unit compares the actual map image to a reference map, to then calculate which region of the foraminous structure requires cleaning - both the cross-directional positioning and - upon considering the speed at which the foraminous structure travels - machine directionally to then actuate the cleaning operation selectively by time and position.

Referring now to Fig. 1A to C, the principle of the present invention is explained without intending to limit the invention to such executions.

Fig. 1A depicts an x-z-directional view of an equipment 1000 as can be operated for the manufacturing of a structure 100 comprising fibers and optionally particles. An air-laying apparatus 1100 exhibits machine direction MD 12, cross-machine CD direction 18 and a z-direction 15 perpendicular to MD and CD. The air-laying unit comprises a material deposition unit 1101, as may be a forming box as well-known from web forming equipment. Further, a first material supply unit may deliver a first material, such as fibers 130 or filaments forming fibers, from a fiber supply unit 1110. Optionally a second material supply unit, as may preferably be a particulate material supply unit 1700 may deliver particles 118 into the material deposition unit 1101 in the material deposition region 1109. As indicated by dashed arrow, the particulate material may alternatively be deposited onto the formed web 100.

The equipment 1000 further comprises a material collection system 1500, comprising a closed loop air permeable collector 1510, preferably a foraminous belt or a foraminous drum, which is continuously moving along the collector path 13. The collector exhibits a material receiving surface 1511, which is oriented such that the materials can be deposited thereon in the material receiving region 1109 of the equipment, and an opposite surface 1519. The collector may be a permeable belt, optionally comprising a shaped mould and/or varying permeability properties to direct the air flow and the material suspended therein to create varying basis weights or densities of the deposited material. A preferred execution of the collector is a foraminous belt, as may be made of
- polyester, polyamide, or with metals like steel or bronze,
- optionally coated,
- preferably dual ply,

exhibiting typically an equivalent circular aperture diameter of
   - less than about 5 mm, or less than 2 mm,
   - but more than about 0.5 mm,
and typically an open area of
   - less than about 40% or less than about 20%,
   - but more than about 10%,
allowing an air flow e.g., at 0.5" of water column or about 124 Pa of
   - more than about 300 cfm about 8.5 m³/min, or more than about 600 cfm or about 17 m³/min, or even more than 1000 cfm or about 23 m³/min,
   - but less than about 2000 cfm or about 56 m³/min.

Various suitable collector belts 1510 are available from Gebr. Kufferath AG (GKD), Germany, or from Nippon Filcon KK, Tokyo, JP, e.g., as a two layer, four shaft fabric with about 1000 cfm (23m³/min) or from Albany Int. Corp, NH, US, under the trade designation Monostat or Microstat.

The collector 1510 is moving along the machine direction 12 through the material deposition region 1109, where the material(s) is/are laid down, by a set of drive and guide rolls 1520.

Thereby, the collector 1510 is moving in a continuous closed loop along the processing direction 13 of the equipment 1000 and thus transferring the structure of deposited materials 100, preferably in a web form comprising fibers, which is formed in the material deposition region 1109 on the collector 1510, towards a further downstream processing unit 1800.

The material collection unit 1500 further comprises a vacuum suction unit 1550 positioned at least in the fiber deposition region 1109 opposite to the material deposition unit 1101, providing an air flow through the collector 1510 from the material receiving surface 1511 to the opposite surface 1519. On the path towards further processing steps 1800, the deposited material 100 may optionally be compacted in a compaction unit, e.g., between two compaction roller 1610.

A collector cleaning device 4000 comprises at least one air blast system 4005 adapted to blow pressurized air through the air permeable collector 1510 in a direction from the opposite surface 1519 towards the material receiving surface 1511 in the cleaning region 4002, which is free of the formed web 100. For the current invention, it is important that the air blast system applies the air blast only when and where necessary.

In Fig. 1B, an air blower system 4005 is depicted in an y-z-directional view. Pressurized air 190 is provided via a flexible connection to a valve unit 4015 connected to one or more, as shown four, nozzles 4017 that are cross-directionally adjacently arranged and further adapted to be moved cross-directionally, e.g., along a slide bar or by a robot arm 4013. Both the opening of the valve and the cross-directional positioning of the combined valve/nozzle unit is controlled by air blast controller 4012, receiving control signals 3290 from the data processing unit 3210 (Fig. 1A), as will be discussed in more detail herein below, that actuate the blowing only cross-directionally where and when the detected clogged region passes the valve/nozzle unit. This allows to provide effective cleaning pulses only when needed, thereby reducing the amount of required pressurized air significantly.

Optionally, the removed material can be captured by a receptacle 4090 as may also be supported by a slight vacuum (not shown).

Fig. 1C depicts a similar system, wherein compressed air 190 is supplied via an air plenum 4019 to multiple valves 4015', 4015", ... with corresponding nozzles 4017, which are positioned cross-directionally adjacently, thus covering the full width of the collector 1510, whereby each of the valve/nozzle units is blowing air only where and when the detected clogged region passes the valve/nozzle unit.

In the exemplary execution as depicted in Fig 1C, four nozzles are connected to one valve, though there can more or less nozzles be combined to a unit - both for the cross-directionally movable unit, or for the cross-directionally adjacent arranged units.

There is not particular restriction to the type and design of the nozzles, which can be circular, or cone or slit-type nozzles.

The air blast activation system and/ or the nozzle positioning systems may comprise stepper motors, pneumatics positioners, or flow diverters. Preferably they comprise closed loop servo motors, exhibiting high dynamics, low response time, preferably of less than 10 ms, and low positioning error. Optionally, they may operate according to pre-set and programmable movement or velocity profiles. Preferably, the PLC units apply a proportional-integral-derivative (PID) control logic, optionally as cascading PID control, e.g., a first level PID controlling deviations resulting from the overall process speed, a second level controlling feed or positioning deviations.

Referring again to Fig. 1A, the equipment according to the present invention further comprises a contact free inspection system 3000 in the inspection region 3010, which is free of the formed web 100, comprising an electromagnetic wave EMW receiver unit 3100, comprising at least one EMW detector 3110, which is arranged over at least one of the surfaces 1511, 1519 of the collector 1510 in a line or a 2D-matrix, or which is cross-directionally movable over at least one of the surfaces 1511, 1519 of the collector 1510. A detector 3110 has a predetermined pixel resolution size that is less than about 10 mm by 10 mm, preferably less than 5 mm by 5 mm, more preferably less than 2 mm by 2 mm. The at least one EMW detector 3110 is adapted to receive EMWs reflected from the surface 1511 or penetrating through the collector 1510, and to convert EMWs in the receiver unit 3100 into an electric signal depending on the transparency or reflection of the collector 1510. The detector 3110 is capable to detect EMWs in the frequency range of from 10 Hz to 10¹⁹Hz, preferably in the range
- of visible light of about 10¹⁴ to 10¹⁵ Hz,
- or of 10¹¹ to 10¹² Hz,
- or of 3x10³ Hz to 3x10⁹ Hz.

In a first particular execution, the wave frequency is in the range of visible light, and conventional video analysis systems can be employed, with suitable systems being available from e.g., Cognex Inc, Natick, MA, USA, or Mitsubishi Electric Europe B.V., Germany, under the trade designation Line Scan Bar. In a second alternative execution, the wave frequency is operated at a range of more than 100 GHz, preferably more than 200 GHz, but less than 900 GHz, preferably less than 600 GHz, most preferably in the range of between 300 GHz and 500 GHz. Suitable detection systems for this execution are disclosed in the above referenced publication WO2018/204724 (Alcatera), to which express reference is made as far as the detection systems are concerned, whilst similar systems are manufactured by Terasense Group Inc. San Jose, CA, USA. In a third alternative execution, the wave frequency is 3x103 Hz to 3x109 Hz, for which system may be available from Accusentry, Marietta, GA, USA. The detector system 3100 should be able to capture the signals from the detectors 3110 at a sufficiently high sampling rate, preferably of at least 24 kHz or more.

Optionally at least one EMW emitter unit 3120 provides EMWs, preferably in the preferred ranges for the detector, to be received by the detectors 3110. The emitter unit 3120 may comprise at least one essentially punctiform emitter of a typical diameter of typically less than 10 mm, or less than 5 mm or even smaller, or a linear or two-dimensional array of such punctiform emitters.

A data processing unit 3200 is adapted to convert the electric signal 170, as received via connection 3190 from the EMW receiver unit 3100, into signals transmitted via connection 3290 to adjust at least one of the processing parameters of the collector cleaning device 4000 by comparing the signal of EMW receiver unit 3100 to a predetermined signal matrix, as may be stored in a data storage unit 3260, which may be further controlled by a human interface device 3250, which may also provide a visible map of the belt over time and position. Thereby, the mapping of the regions along the length of the collector 3150 is correlated via the speed 13 of the collector 1510, as may monitored by collector speed detector 3270, transmitting the speed signal to the data processing unit via connection 3280. It should be noted that any connections may be via cable or wireless or other data communication channels.

Thus, the air blast system 4005 of the collector cleaning device 4000 is adapted to provide air blasts upon activation of the air blast control devices 4012 by the data processing unit 3200, wherein the air blasts are discrete over time, i.e., non-permanent, and position, i.e., not over the full width relative to the collector.

In case that for the moveable nozzle systems (see Fig. 1B) two or more regions on the same cross-directional position were detected, the system should be able to store these positions for a subsequent loop of the collector 1510. For continuing deviations, an alarm may alert an operator, or the system may automatically stop.

Thus, the computer implemented method to control the cleaning of a web forming system comprising a movable foraminous material collector comprises the recuring steps of
- creating continuously an image of the collector, which is mapping a pattern of reflected or penetrated EMWs;
- comparing the image to a predetermined image map of the collector and determining a deviation pattern, mapping the deviations cross-directionally and along the collector length by taking into account the speed of the collector;
- calculating when and where the air blast system needs to be actuated to create a cleaning air blast;
- actuating the air blast system.

## Claims

1. An equipment (1000) for inspecting and cleaning a foraminous collector (1510), preferably a foraminous belt for collecting fibrous (130) and optionally particulate material (118),
said equipment (1000) comprising
1) an air-laying apparatus (1100) exhibiting machine machine-direction MD (12), cross-machine direction CD (18) and a z- or height direction (15) perpendicular to MD and CD, said air-laying apparatus (1000) comprising
1a) a first material supply unit, preferably a fiber supply unit (1110) for supplying fibers (130) or fiber forming filaments, and optionally a second material supply unit, preferably a particulate material supply unit (1700);
1b) a material deposition unit (1101), preferably a forming box, defining a deposition region (1109);
2) a material collection unit, comprising
2a) a closed loop air permeable collector system (1500), comprising
2a1) a continuously moving foraminous collector (1510), preferably a foraminous belt;
2a1i) comprising a material receiving surface (1511) and an opposite surface (1519),
2a1ii) and moving machine directionally (12) through said material deposition region (1109);
2a2) said collector system (1500) comprising a set of drive and guide rolls (1520) adapted
2a2i) to move said collector (1510) in a continuous closed loop along the processing direction (13) of said collector (1510) at a collector speed, preferably monitored by a collector speed detector (3270), and
2a2ii) to transfer a web (100) formed in said material deposition region (1109) on said collector (1510) to a further downstream processing unit (1800);
2b) a vacuum suction unit (1550) positioned in said fiber deposition region (1109) opposite to said material deposition unit (1101) and being adapted to provide an air flow through said collector from said material receiving surface (1511) to said opposite surface (1519);
2c) optionally a compaction unit (1610) for compacting said web (100);
3) a collector cleaning device (4000) comprising
3a) at least one air blast system (4005) adapted to blow air through said air permeable collector (1510) in a direction from said opposite surface (1519) towards said material receiving surface (1511);
3b) an air blast system control device (4012) adapted to adjust time and CD-position of air blown through said collector (1510);
4) a contact free inspection unit (3000) comprising
4a) electromagnetic wave (EMW) receiver unit (3100), comprising
4a1) at least one EMW detector (3110), which is
4a1i) arranged
- over at least one of said surfaces (1511, 1519) of said collector (1510) in a line or a 2D-matrix over said collector,
or
- movably cross-directionally over at least one of said surfaces (1511, 1519) of said collector (1510);
4a2) wherein said at least one EMW detector (3110) being adapted 4a2i) to receive EMWs reflected from the surface (1511, 1519) of or penetrating through said collector (1510), and
4a2ii) to convert EMWs in the range of from 10 Hz to 10¹⁹Hz, preferably in the range
- of visible light of about 10¹⁴ to 10¹⁵ Hz,
- or of 10¹¹ to 10¹² Hz,
- or of 3x10³ Hz to 3x10⁹ Hz,
into an electric signal (170) depending on the transparency or reflection of said collector (1510);
4b) optionally at least one EMW emitter unit (3120), providing EMWs detectable by said detector(s) (3110);
4c) a data processing unit (3200) adapted to convert the said electric signal of said inspection system into signals for adjusting at least one of the processing parameters of said collector cleaning device (4000) by comparing the said electric signal to a predetermined signal matrix;
wherein
- said collector cleaning device (4000) and said EMW receiver unit (3110) are positioned in a region that is free of said web (100) formed on said collector (1510);
- and wherein said air blast system (4002) of said collector cleaning device (4000) is adapted to provide air blasts upon activation of said air blast control device (4012) by said data processing unit (3200), wherein said air blasts are discrete over time and position relative to the collector.

2. An equipment (1000) for inspecting and cleaning a foraminous collector (1510) according to claim 1, wherein said detector (3110) is oriented towards said receiving surface of said collection (1510).

3. An equipment (1000) for inspecting and cleaning a foraminous collector (1510) according to claim 1 or 2, wherein said cleaning system comprises a multiplicity of cross-directionally arranged valves (4015) with nozzle (4017) units, which are adapted to be individually addressable.

4. An equipment (1000) for inspecting and cleaning a foraminous collector (1510) according to claim 1 or 2, wherein said cleaning system comprises a valve (4015) with nozzle (4017) unit which is adapted to be cross-directionally movable.

5. A process for inspecting and cleaning a foraminous collector (1510), preferably a foraminous belt for collecting fibrous (130) and optionally particulate material (118), said process comprising the steps of
A) providing an equipment according to any of claims 1 to 4;
B) inspecting said collector (1510) in said inspection region (3010) by said contact-free inspection unit (3000) by receiving EMWs by said at least one receiver unit (3110), said inspecting comprising the steps of
B1) receiving a time and position depending signal (170) as a function of the collector's reflection or transparency for the EMWs,
B2) transmitting said signal (170) to said data processing unit (3200);
C) analysing said signal (170) in said data processing unit (3200)
C1) by comparing to a pre-determined set of collector's transparency or reflection parameters,
C2) and determining the position of an off-target collector region of said collector (1510);
D) providing a signal for actuating said collector cleaning device depending on the time and position dependent variations;
E) moving said off-target collector region towards said collector cleaning device;
G) actuating said collector cleaning device so as to create an air blast at a time and position corresponding to the off-target collector region,
wherein steps B) to G) are executed in a continuous operation.

6. A computer implemented method to control the cleaning of a web forming system comprising a movable foraminous material collector (1510) comprising the recuring steps of
- creating an image of the collector, which is mapping a pattern of reflected or penetrated EMWs;
- comparing the image to a predetermined image map of the collector and determining a deviation pattern, mapping the deviations cross-directionally and along the collector length by taking into account the speed of the collector;
- calculating when and where the air blast system needs to be actuated to create a cleaning air blast;
- actuating the air blast system.
